Europäisches Patentamt

European Patent Office

Office européen des brevets

(11) Publication number: **0 496 453 A1**

## (12) EUROPEAN PATENT APPLICATION

(21) Application number: **92200112.8**

(22) Date of filing: **16.01.92**

(51) Int. Cl.5: **C12N 15/85**, C12N 15/62, C12N 15/12, C07K 15/00

(30) Priority: **23.01.91 US 644801**

(43) Date of publication of application:
**29.07.92 Bulletin 92/31**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Berger, Joel P.**
**9 Fairfax Drive**
**Livingston, NJ 07039(US)**

(74) Representative: **Thompson, John Dr. et al**
**Merck & Co., Inc. European Patent**
**Department Terlings Park Eastwick Road**
**Harlow, Essex CM20 2OR(GB)**

(54) **Human glut4 gene promoter.**

(57) A unique DNA sequence associated with the expression of the human glut4 glucose transporter of mammalian cells has been isolated, purified, cloned and sequenced. This DNA sequence is used to regulate the expression of genes encoding proteins of interest in cells from selected tissues.

FIG. 3

## BRIEF DESCRIPTION OF THE DRAWINGS

FIGURE 1. Amplification strategy showing that the promoter region must be adjacent to the 21 kb vector arm containing the T7 promoter.

FIGURE 2A. Nucleotide sequence for the human glut4 promoter.

FIGURE 2B. Nucleotide sequence for the human glut4 promoter.

FIGURE 3. Plasmid containing human glut4 promoter and an indicator gene.

## BACKGROUND OF THE INVENTION

The uptake of glucose by cells is mediated by a family of five glucose transport proteins that have been shown to be expressed in a tissue-specific manner (for review see Bell et al. Diabetes Care 13: 198-208 [1990]; Mueckler, Diabetes: 39: 6-11 [1990]). One member of the family, glut4, is expressed specifically in skeletal muscle, adipose and heart (Birnbaum, Cell 57: 305-315, [1989]; Charron et al., Proc. Natl. Acad. Sci. USA 86: 2535-2539 [1989]; Fukumoto et al., J. Biol. Chem. 264: 7776-7779 [1989]; James et al., Nature 388: 83-87 [1989]), and appears to be the major transporter isoform responsible for insulin-induced glucose uptake by these tissue (Berger et al., Nature 340: 70-72 [1989]; Garvey et al., Science 245: 60-63 [1989]; Kahn et al., J. Clin. Invest. 84: 404-411 [1989]; Sivitz et al., Nature 340: 72-74 [1989]; Strout et al., Endocrino. 126: 2728-2732 [1990]; and Zorzano et al., J. Biol. Chem. 264: 12358-12363 [1989]).

Recently, a decrease in the expression of glut4 and its messenger RNA was observed in animal models of insulin resistance (Berger et al., Nature 340: 70-72 [1989]; Garvey et al., Science 245: 60-63, [1989]; Kahn et al., J. Clin. Invest. 84: 404-411 [1989]; Sivitz et al., Nature 340: 72-74 [1989]; Strout et al., Endocrino. 126: 2728-2732 [1990]). A decrease in skeletal muscle adipose glut4 expression was also observed in morbidly obese diabetics (Caro et al., Diab. Metab. Rev. 5: 665-689 [1989]) while significant diminution was not seen in lean and mildly obese diabetics (Pedersen et al., Diabetes 39: 865-870 [1990]). This evidence suggests that diminished expression of glut4 at the pretranslational level may play a role in insulin resistance and subsequent hyperglycemia observed in some patients suffering from NIDDM.

The murine glut4 gene has recently been cloned and characterized (Kaestner et al., Proc. Natl. Acad. Sci. USA 86: 3150-3154 [1990]). Its promoter was shown to possess a specific binding site for C/EBP and to be trans-activated by this protein in 3T3-LI cells. The present invention discloses the cloning of the unique human glut4 5'-flanking region utilizing PCR amplification and hybridization techniques. Sequence analysis of this region demonstrates the existence of an appropriately placed CCAAT box and putative MyoD, C/EBP, IRE-A, and SPI binding sites.

At least two sequence elements are required for the regulation of genes that encode messenger RNA in mammalian cells: promoters and enhancers. Promoters are located upstream from the start site of transcription and are generally about 100 base pairs in length, Dynan and Tjian, Nature 316: 774 (1985) and McKnight and Tjian, Cell 46: (1986). A promoter is required for accurate and efficient initiation of transcription, while enhancers increase the rate of transcription form promoters. Enhancers are characterized by their ability to stimulate transcription, from both homologous and heterologous promoters, Voss et al., TIBS 11: 287 (1986). Enhancers appear unique in that they can act on cis-linked promoters at great distances in an orientation-independent manner and can also function downstream from the transcription unit, Serfling et al., Trends. Genet. 1: 224(1985). In many situations there may no clear division between enhancer and promoter sequences because certain sequences with promoter activity in one situation can be important as a functional enhancer in another. Moreover, tandem copies of some promoter sequences can acquire properties of enhancers, suggesting that it is the context of a given sequence that determines its functional characteristic rather than them being an intrinsic property of the binding protein, La Tangue and Rigby, pg. 6, in Transcription and Splicing, Hames and Glover, Eds., IRL Press, Washington, D.C., 1988.

Some of the cis-linked promoters, such as the TATA box, are shared by many promoters and bind general transcription factors. Other promoters, such as SP1 binding sites and the CCAAT box, are found in selected promoters, La Thangue and Rigby, pg. 6, in Transcription and Splicing, Hames and Glover, Eds., IRL Press, Washington, D.C., 1988. The promoter/enhancer systems may be further regulated by the binding of specific proteins or polypeptides to segments of the promoter/enhancer system. Indeed, insulin may function in the regulation of the glucose transport proteins.

## OBJECTS OF THE INVENTION

It is, accordingly, an object of the present invention to isolate and purify the promoter region of the human glut4 glucose transporter. Another object is to provide for tissue specific expression of heterologous genes with the novel glut4 promoter region.

## SUMMARY OF THE INVENTION

A unique DNA sequence associated with the expression of the human glut4 glucose transporter of mammalian cells has been isolated, purified, cloned and sequenced. This DNA sequence is used to regulate the expression of genes encoding proteins of interest. The DNA sequence is operably attached to a gene for a desired protein and incorporated into a vector. The novel vector is used to transform a eukaroytic cell host such that the promoter regulates expression of the gene for the desired protein.

## DETAILED DESCRIPTION OF INVENTION

The present invention relates to the isolation, purification, characterization and sequencing of the mammalian glut4 promoter DNA sequence which can be used to regulate the expression of DNA encoding proteins in mammalian cells. Promoter as used herein is defined as a DNA sequence which includes not only one or more promoter DNA sequences but may also include one or more enhancer sequences. The sequences are collectively referred to as regulatory sequences. Since there may no clear division between enhancer and promoter sequences and because tandem copies of some promoter sequences can acquire properties of enhancers, the term promoter is intended to include both elements. Promoter as used herein is also defined as a nucleotide sequence or sequences found upstream of a gene that act as a signal for the binding of RNA polymerase. Like promoters, enhancers consist of short arrays of DNA sequences that interact specifically with cellular proteins involved in transcription. The combination of different recognition sequences and the amounts of the cognate transcription factors determine the efficiency with which a given gene is transcribed in a particular cell type. This invention also includes the utilization of this unique promoter for the production of polypeptides and proteins encoded by heterologous genes operably attached to this promoter. Heterologous gene as used herein refers to a structural gene or genes other than the glut4 glucose transproter gene, and structural gene refers to a nucleotide sequence which may be transcribed and translated by a cell to produce a polypeptide chain. Operably attached is defined as the orientation of various DNA segments which will result in the transcription and translation of a gene of interest. The invention also includes the use of the unique promoter for the specific expression of operably attached heterologous genes in skeletal muscle cells, adipose cells and dermal fibroblasts and other cell types with similar specificities. It is intended that the present invention include all promoter DNA sequences for the Glut4 glucose transporter gene and any and all enhancer DNA sequences associated with said promoter which allow the expression of a heterologous gene. The arrangement of the regulatory sequences maybe that found in the DNA sequence or in any arrangement which will allow the transcription and translation of a heterologous gene operably attached to the glut4 promoter.

The promoter is isolated from a human placental genomic DNA library contained in the EMBL-3 SP6/T7 vector. The genomic library can be obtained commercially from Clontech (Palo Alto, CA). It is to be understood that other genomic libraries can be used to isolate and purify promoters for facilitative glucose transporter proteins. It is further understood that an appropriate promoter can be isolated and purified from any human cell capable of expressing the glut4 transporter gene, since the promoter is genomic it can be isolated from any human cell. The genomic library DNA is purified and the library phage stock amplified by the following process. About $1 \times 10^6$ plaque forming units (pfu) of library phage in about 10 $\mu$l of about 50 mM Tris-HCl, pH about 7.5, containing about 100 mM NaCl, about 8 mM MgSO$_4$ and about 0.01% gelatin is combined with about 100 $\mu$l of about 10 mM MgSO$_4$, about 10 mM CaCl$_2$ and about 75 $\mu$l log phase LE392 E. coli in Luria Bertani (LB) broth containing about 0.2% maltose and about 10 mM MgSO$_4$. The phage are allowed to preabsorbe to the cells for about 30 minutes at 37° C, after which about 6 nil of LB broth is added and the cells are incubated at about 37o C for about 16 hr rotating at about 225 revolutions per minute (rpm). This procedure is repeated about 26 times to amplify a total of about $2.6 \times 10^7$ pfu. Alter incubation, the lysates are pooled and cell debris removed by centrifugation (about 10 minutes at about 8,000 x g). The library phage DNA is isolated using Lambdasorb Phage Adsorbent in a protocol provided by the manufacturer (Promega).

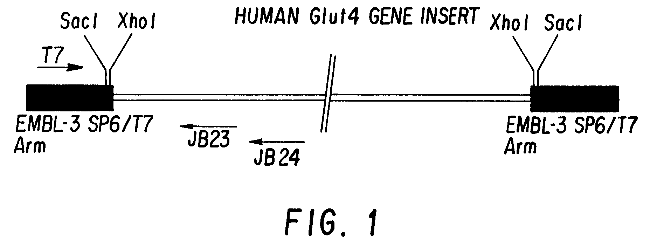

In order to obtain template DNA for the polymerase chain reaction amplification of the 5' promoter region of the human glut4 gene, it was determined that $2.6 \times 10^7$ pfu of phage stock from the human genomic DNA library in vector EMBL3 SP6/T7 would be necessary. This is greater than 10 times the calculated number of independent plaques necessary to be screened by standard hybridization methods in order to have a 99% probability of obtaining the clone of interest. The increased number of clones utilized here is necessitated by an amplification strategy which will only succeed on templates in which the promoter region is adjacent to the 21 kilobase (kb) vector arm containing the T7 promoter (Fig. 1). Since it was desirable to amplify a fragment of approximately 1 kb and the average insert size is about 15 kb, calculations determined that only about 1 in 15 or about 7% of the phage DNA containing the promoter would serve as a proper template. In order to maintain a high probability of amplifying the desired sequence, the number of plaques "screened" is therefore increased by a factor of 10 or more. The 2.6 x $10^7$ pfu are amplified in 26 separate 6 ml liquid lysates yielding a total of greater than 1 x $10^{12}$ pfu. The Lambdasorb purification results in about 50 $\mu$g of high molecular weight template DNA.

Oligonucleotide primers are prepared for polymerase chain reaction amplification of the glut4 promoter gene. Oligonucleotide primers are synthesized on an Applied Biosystems Model 8700 automated DNA synthesizer and purified on a SEP-PAK C18 cartridge (Waters Assoc.) as described by the manufactures. The oligonucleotide primers are determined from a nucleotide sequence upstream from the sequence of human adult skeletal muscle glucose transporter as shown by Fukumoto et al., J. Biol. Chem. 264: 7776-7779 (1989) and from the nucleotide sequence of the T7 promoter region. Three different primers are used:

Primer T7, 22mer: 5' TAATACGACTCATATAGGGGAG 3' (SEQ ID NO: 1)
Primer JB23, 21mer: 5' GATCCTGGAGTCTCTGACTCC 3' (SEQ ID NO: 2)
Primer JB24, 22mer: 5' GCGAAGATGAAAGAACCGATCC 3' (SEQ ID NO: 3)

Primer T7 contains the nucleotide sequence of the T7 promoter located in the 21 kilobase arm of the EMBL-3 SP6/T7 vector. Primers JB23 and JB24 contain sequence complimentary to nucleotides 77-97 and 92-113, respectively, of the human glut4 cDNA positive strand as described by Fukumoto et al., J. Biol. Chem. 7776-7779 (1989). It should be noted that the above primers are illustrative of primers which may be used for the amplification of the glut4 promoter region, but others may be identified and used in the following process.

The first round of amplification is performed in about 100 $\mu$l of 1 x PCR buffer ( about 10 mM Tris-HCl, pH about 8.3, about 50 mM KCl, about 1,5 mM $MgCl_2$, about 0.01% (w/v) gelatin) containing about 200 $\mu$M dNTP's, about 100 picomoles each of primers T7 and JB24, and about 250 ng of the above mentioned human genomic library DNA as template. The reaction mixture was overlayed with about 100 $\mu$l of mineral oil. The mixture was preincubated at about 94° C for about 8 minutes and then rapidly cooled to 55° C in a DNA thermal cycler (Perkin-Elmer, Cetus). Taq polymerase, about 1,0 units, (Perkin-Elmer, Cetus) is added and the reactions are carried out for 35 cycles. Each cycle contains a heat denaturation step at about 94° C for about 2 minutes, followed by annealing of the primers to the cDNA at 55° C for about 2 minutes, and DNA chain extension at about 72° C for about 1 minute. Following amplification, the incubation mixture is extracted one time with chloroform prior to further processing. This initial amplification produces a detectable DNA fragment of about 50 bp in length. It was determined that the polymerase chain reaction preferentially amplified shorter template fragments along with a few larger fragments. The larger fragments could not be detected by ethidium bromide staining of an agarose gel.

In order to further amplify a larger section of the promoter region, a second round of amplification is carried out using the process of the first amplification. The second round of amplification is performed in the same manner as the first except the primers utilized are T7 and JB23 and the template is 100 nl of the first round amplification reaction mixture. The later "nested" oligonucleotide contains glut4 cDNA sequence found upstream and adjacent to that found in JB24. Analysis of the second reaction mixture by agarose gel electrophoresis reveals the amplification of a DNA fragment of about 450 bp.

The promoter region obtained in the second round of amplification is purified by agarose gel electrophoresis, blunt-ended with Klenow DNA polymerase I, phosphorylated utilizing polynucleotide kinase and subcloned into the SmaI site of the vector SP72 (Clontech). Three independent clones are identified and sequenced by the method of Sanger et al., Proc. Natl. Acad. Sci. USA 74: 5463-5467 (1977). The inserts in the 3 independent clones are sequenced and are identical. The nucleotide sequence found in triplicate contains nucleotides -314 to +142 as presented in the sequence of a larger second clone subsequently isolated from the same library by standard hybridization techniques, see Figure followed by annealing of the primers to the cDNA at 55° C for about 2 minutes, and DNA chain extension at about 72° C for about 1 minute. Following amplification, the incubation mixture is extracted one time with chloroform prior to further processing. This initial amplification produces a detectable DNA fragment of about 50 bp in length. It was determined that the polymerase chain reaction preferentially amplified shorter template fragments along with a few larger fragments. The larger fragments could not be detected by ethidium bromide staining of an agarose gel.

In order to further amplify a larger section of the promoter region, a second round of amplification is carried out using the process of the first amplification. The second round of amplification is performed in the same manner as the first except the primers utilized are T7 and JB23 and the template is 100 nl of the first round amplification reaction mixture. The later "nested" oligonucleotide contains glut4 cDNA sequence found upstream and adjacent to that found in JB24. Analysis of the second reaction mixture by agarose gel electrophoresis reveals the amplification of a DNA fragment of about 450 bp.

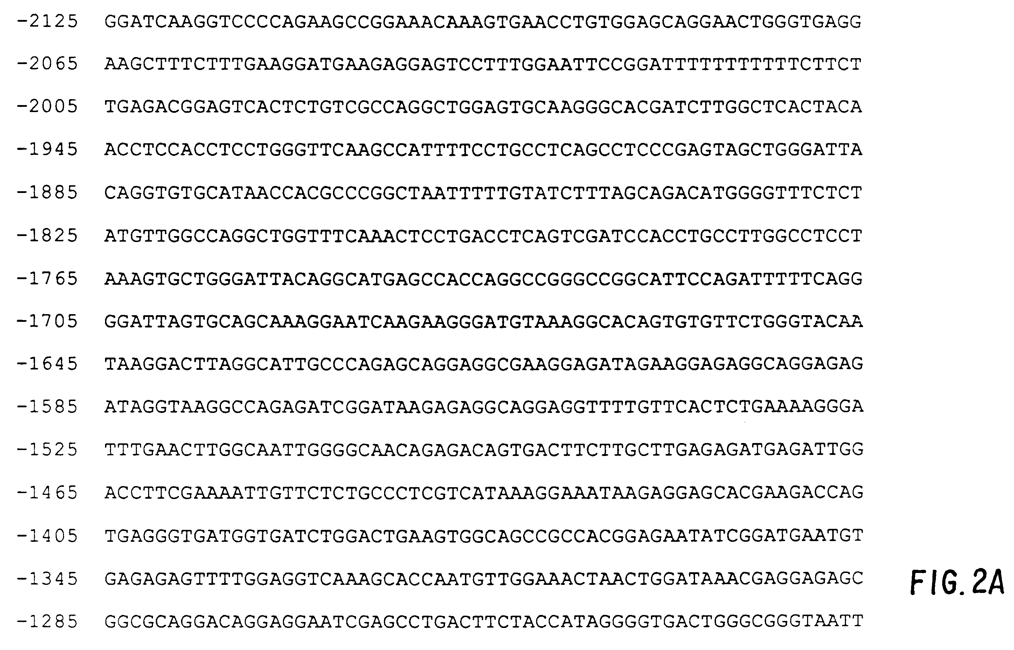

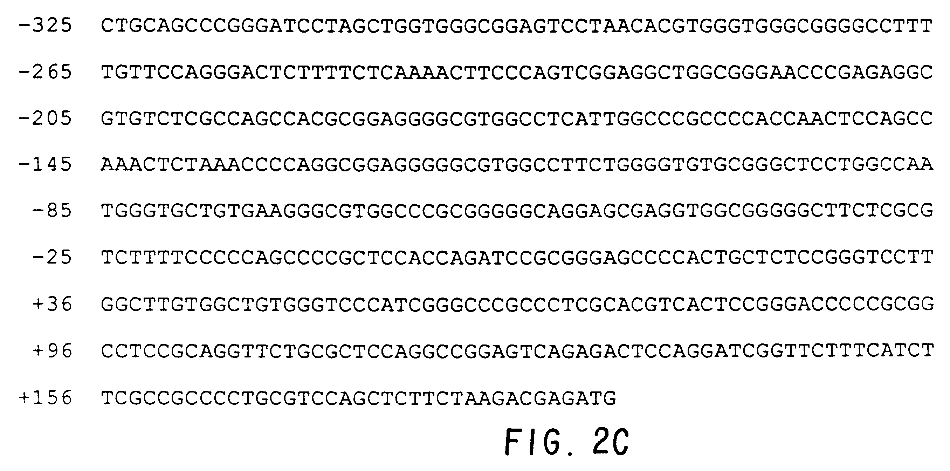

The promoter region obtained in the second round of amplification is purified by agarose gel electrophoresis, blunt-ended with Klenow DNA polymerase I, phosphorylated utilizing polynucleotide kinase and subcloned into the SmaI site of the vector SP72 (Clontech). Three independent clones are identified and sequenced by the method of Sanger et al., Proc. Natl. Acad. Sci. USA 74: 5463-5467 (1977). The inserts in the 3 independent clones are sequenced and are identical. The nucleotide sequence found in triplicate contains nucleotides -314 to +142 as presented in the sequence of a larger second clone subsequently isolated from the same library by standard hybridization techniques, see Figure 2A snd 2B (SEQ ID NO: 4). The amplified fragment is about 458 bp in length and extends from 50 to 508 nucleotides upstream of the initial methionine codon fo the human glut4 cDNA.

The glut4 promoter/enhancer sequence is operably attached to a heterologous gene which encodes a specific heterologous protein and the promoter-gene construct is used to transfect cells. The promoter/enhancer sequence can be used to express a multitude of polypeptides and proteins, including for example, enzymes, growth factors, lymphokines, monokines and reporter proteins as exemplified by the listing in U.S. Patent Serial No. 4,935.363 and in Beaudet, Am. J. Hum. Gen., 37: 386-406(1985). It is to be understood that most proteins could be expressed by recombinant technology using the glut4 promoter system and the cell types described above.

The glut4 promoter region of the present invention is used to control expression of genes or DNA sequences operably attached to and subsequent to or down stream from said promoter. Indeed, this promoter when located upstream to a selected heterologous structural gene will result in the expression of that gene in an acceptable host cell. Upstream as used herein refers to DNA sequences found in a 5' direction from a given point of reference along a DNA molecule. Heterologous or structural gene relates to DNA sequences that are not normally associated with the glut4 promoter region and can be both transcribed and translated by a host cell. This is illustrated by removing the glut4 promoter region from the SP72 vector by cleavage within the polylinker region of the vector with restriction endonucleases BamHI and EcoRI. The excised glut4 promoter region is purified by agarose gel electrophoresis blunt-ended with Klenow DNA polymerase I and subcloned into the blunt-ended BamHI site of a vector containing a structural or heterologous gene. The promoter sequence of the present invention may be used for the transcription and translation of any heterologous gene, with or without additional homologous or heterologous control or promotional sequences.

The glut4 promoter can also be used for the expression of reporter or indicator genes which allow quantitative determination of gene expression. Attachment of these genes to a promoter followed by introduction of the DNA into eucaryotic cells permits the indirect determination of the effect of that operably attached sequence on the level of gene expression. Indicator genes include, but are not limited to, E. coli $\beta$-galactosidase (An et al., Nucleic Acids Res. 13: 2921-2930 [1985], galactokinase ( Schumperli et al., Proc. Natl. Acad. Sci. USA 79: 257-261 [1982], interleukin-2 (Cullen, Cell 46: 973-982 [1986], thymidine kinase (Searle et al., Mol. Cell. Biol. 5: 1480-1489 [1985], and secretable alkaline phosphatase (European Patent Application, Publication No. 327,960), with alkaline phosphatase being preferred.

The blunt ended glut4 promoter is, for example, subcloned into a blunt ended- BamHI site of a vector containing secretable alkaline phosphatase (SEAP), such as pSVO/SEAP. The vector pSVOSEAP is constructed from the pSVOAP vector described by Yoon et al., Gene 66: 11-17 (1988) and the pBC12/RSV/SEAP vector described by Berger et al., Gene 66: 1-10 (1988). The vector pSVOAP is cut with the restriction enzymes Sma I and Hind III to remove the rat alkaline phosphatase cDNA from the vector. The remaining vector pSVO is pruified by electrophoresis in an agarose gel. The vector pBC12/RSV/SEAP is cleaved with the restriction enzyme Xho I, filled in with Klenow fragment and cut with Hind III to remove the cDNA encoding secreted alkaline phosphatase (SEAP). The SEAP cDNA is purified by electrophoresis in an agarose gel. The SEAP cDNA is ligated into the Sma I/Hind III site of pSVO to yield pSVOSEAP. A clone was selected in which the orientation of the glut4 promoter region was appropriate for the expression of a gene located down stream from the promoter. This clone is designated pM/AGT/SEAP. A second clone with the glut4 promoter region in the opposing orientation was designated pM/AGT(REV)/SEAP. The constructs are transfected into cells, generally mammalian cells and cell lines, by a know process such as calcium phosphate coprecipitation and secreted reporter gene activity of the media is determined at about 72 h post-transfection. Cells should be stable and relative easy to grow in large numbers and should contain as little native background as possible of the reporter gene or gene product. Mammalian cells and cell lines may be limited to cells from tissues, skeletal muscle, heart and adipose, which generally express glut4 glucose transport protein or cells modified to express proteins under control of the glut4 promoter. The cell lines include, but are not limited to,3T3-L1 and CHO. Cells transfected with pM/AGT/SEAP produced about 10 times more alkaline phosphatase activity than those transfected with an equal amount of pM/AGT (rev)-/SEAP.

The following examples illustrate the present invention without, however, limiting the same thereto.

EXAMPLE 1

Isolation, Purification and Characterization Of Human Glut4 Gene Promoter

Purification of genomic library DNA.

To obtain template DNA for the polymerase chain reaction amplification of the 5' promoter region of the human glut4 gene it was determined that approximately $2.6 \times 10^7$ pfu of phage stock from a human genomic DNA library in vector EMBL3 SP6/T7 would be necessary. This is approximately 20 times greater than the calculated number of independent plaques necessary to be screened by standard hybridization methods in order to have a 99% probability of obtaining the clone of interest. It was assumed that a fragment of about 1 kb could be amplified from a template in which the glut4 5'-flanking region was adjacent to the 21 kb vector arm (Figure 1). Since the average insert size was about 15 kb, it was further assumed that only 1/15 or about 7% of the phage DNA containing the 5' region would have the sequence complementary to JB24 within 1 kb of the T7 promoter and therefore serve as a proper template. To maintain a high probability of amplifying the desired sequence, the number of plaques "screened" was therefore increased.

The human placental genomic DNA library in vector EMBL-3 SP6/T7 was obtained as a phage stock from Clontech (Palo Alto, Cal.). Library phage stock was amplified in the following manner. Plaque forming units (pfu), $1 \times 10^6$, of library phage in 100 $\mu$l of 50 mm tris-HCl pH 7.5 containing 100 mm NaCl, 8 mm MgSO$_4$ and 0.01% gelatin was combined with 100 $\mu$l of 10 mM MgSO$_4$, 10 mM CaCl$_2$ s and 75 $\mu$l log phase LE392 E. coli in Luria Broth containing 0.2% maltose and 10 mM MgSO$_4$. The phage were allowed to preadsorb to the cells for 30 min at 37° C, 6ml of LB was added and incubation was allowed to continue at 37° C for 16 hours at 225 rpm. This procedure was repeated 26 times allowing the amplification of $2.6 \times 10^7$ pfu. Amplification yielded a total of greater than $1 \times 10^{12}$ pfu. After incubation the lysates were pooled and cell debris was removed by centrifugation (10 min at 8,000 x g). The library phage DNA was then isolated using Lambdasorb Phage Adsorbent in a protocol provided by the manufacturer of the adsorbent (Promega, Madison, Wis.). More than 50 $\mu$g of high molecular weight template DNA was obtained. Polymerase Chain Reaction Amplification of the Glut4 Promoter Region.

Three oligonucleotide primers were synthesized on an Applied Biosystems Model 8700 automated DNA synthesizer and purified on a SEP-PAK C18 cartridge (Waters Associates, Milford, Mass.) as described by the manufacturers. Three different primers were used:

Primer T7, 22mer: 5'TAATACGACTCACTATAGGGAG 3' (SEQ ID NO: 1)

Primer JB23, 21mer: 5' GATCCTGGAGTCTCTGACTCC 3' (SEQ ID NO: 2)

Primer JB24, 22mer: 5' GCGAAGATGAAAGAACCGATCC 3' (SEQ ID NO: 3)

Primer T7 contains a nucleotide sequence found in the T7 promoter located in the 21 kilobase arm of the EMBL-3 SP6/T7 vector. Primer JB23 and JB24 contain sequence complimentary to nucleotides 77-97 and 92-113, respectively of the human glut4 cDNA positive strand (numbering Fukumoto et. al., J. Biol. Chem. 264: 7776-7779 (1989).

The first round of amplification was performed utilizing oligonucleotides T7 and JB 24 and 250 ng of template DNA (see below). It was calculated that this 250 ng aliquot would provide sufficient template DNA form each library plaque so that the desired fragment could be amplified to a detectable level. In fact, the first amplification reaction produced a detectable DNA fragment when examined by agarose gel electrophoresis. The fragment was only about 50 base pairs (bp) in length

The first round of amplification was performed in 100 $\mu$l of 1 x PCR buffer (10 mM Tris-HCl, pH 8.3, 50 mM KCl, 1.5 mM MgCl2, 0.01% (w/v) gelatin) containing 200 $\mu$m dNTPs, 100 picomoles each primers T7 and JB24, and 250 ng of the aforementioned human genomic library DNA as template. The reaction mixture was overlayed with 100 $\mu$l of mineral oil. The mixture was preincubated at 94 C for 8 min and then rapidly cooled to 55°C in a DNA thermal cycler (Perkin-Elmer Cetus). Taq polymerase (1.0 unit, Perkin-Elmer Cetus) was added and the reactions were carried out for 35 cycles. Each cycle contained a heat denaturation step at 94°C for 2 min, followed by annealing of the primers to the cDNA at 55°C for 2 min, and DNA chain extension at 72°C for 1 m. At completion the incubation was extracted one time with chloroform prior to further processing.

The sequence of the human glut4 cDNA 5' untranslated region was reexamined and it was discovered that a Sau 3A restriction site existed approximately 50 bp from the 5' end of JB23. We concluded that the major fragment being produced during the first round of amplification was obtained from a template(s) in which genomic DNA cleaved at this Sau3A site had been ligated into the vector so that the site was adjacent to the T7 promoter region. Since the library was constructed from genomic DNA that had been partially cleaved with Sau 3A it seemed correct to assume that template would also exist that contained DNA cut at sites further upstream in the glut4 promoter region. We concluded that the polymerase chain reaction was preferentially amplifying shorter template fragments but that larger fragments might also be amplified albeit at levels below that detectable by ethidium bromide staining of an agarose gel.

In order to further amplify a larger section of the promoter region, a second round of amplification was performed in the same manner as the first except, an aliquot of the first round amplification was utilized as template and oligonucleotides T7 and JB 23 served as primers. The latter "nested" oligonucleotide is complementary to the glut4 cDNA sequence found upstream and adjacent to that found in JB 24. Analysis of the reaction mixture by agarose gel electrophoresis revealed the amplification of a DNA fragment of about 450 bp.

Subcloning and Sequencing of the Glut4 Promoter Region.

The promoter region obtained in the second round of amplification was purified by agarose gel electrophoresis, blunt-ended with Klenow DNA polymerase I, phosphorylated utilizing Polynucleotide kinase and subcloned into the SmaI site of the vector SP72 (Clontech). Alkali-denatured double stranded DNA from three independent clones were sequenced utilizing [$\alpha$ -35S] dATP and Sequenase (US Biochemicals). The amplified fragment is 458 bp in length and extends form 50 to 508 nucleotides upstream of the initial methionine codon of the human glut4 cDNA.

Southern blot analysis of human genomic DNA.

High molecular weight human placental genomic DNA (Clontech) was digested with BamHI, HindIII, EcoRI or XbaI, size fractionated on a 0.8% agarose gel and transferred to a reinforced nitrocellulose filter (Stratagene) in 20 X SSC. The membrane was prehybridized at 42°C in 5 x SSC, 50% formamide, 1% SDS, 5 x Denhardt's solution and 150 $\mu$g/ml heat-denatured salmon sperm DNA for 4 hours and subsequently hybridized overnight at 42°C in the same buffer containing the PCR-derived human glut4 promoter region. This probe was radiolabeled with $^{32}$P-dCTP (3000 Ci/mM, Amersham) by random priming (Prime time, International Biotechnology, Inc.) to a specific activity of about 2 x 10$^8$ cpm/$\mu$g utilizing the method of the manufacturer. The hybridization was carried out with 2 x 106 cpm/ml hybridization buffer. The filter was finally washed in 0.1 x SSC, 0.1% at 60° C and exposed to Kodak XAR-5 film.

Cloning of glut4 promoter region from human genomic DNA library.

The partial Sau 3A human genomic library utilized above was plated at a density of 50,000-70,000 plaques/150 mm plate and transferred to reinforced nitrocellulose filters for hybridization to the PCR-derived human glut4 promoter region clone as described above. One positive plaque was obtained. Alter two rounds of plaque purification, DNA from the phage was isolated and found by restriction analysis to contain an insert of about 16kb. A 5.2 kb Xho I insert fragment was subcloned into the Xho I site of pBluescript II KS + (Stratagene, Inc). Restriction analysis of this fragment revealed that it contained greater than 2 kb of sequence upstream of the human glut4 cDNA 5'-untranslated region.

Sequence analysis of the human glut4 5'-flanking region

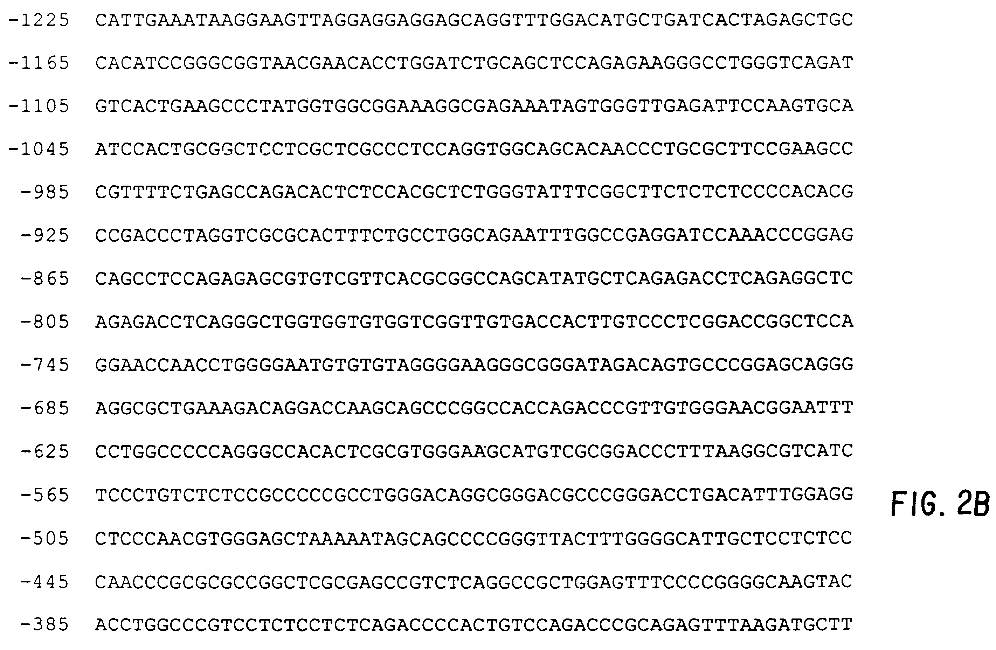

The nucleotide sequence of the human glut4 5'-flanking region is presented in Figures 2A and 2B (SEQ ID NO: 4). It contains the sequence CACCAGATCC which matches 9 of 10 nucleotides found at the transcription start site of the murine glut4 mRNA as determined by Kaestner et al., Proc. Natl. Acad. Sci. USA 86: 3150-3154 (1990). Consequently, this site, the fifth nucleotide in the sequence, has been assigned position +1. The sequence from nucleotide +47 to +189 is identical to the human glut4 cDNA 5'-untranslated region reported by Fukumoto et. al.(1989) with a single exception at nucleotide +48 where the genomic sequence contains a T while the cDNA was reported as having a G. The clone extends 2125 bp upstream beyond the assigned transcription start site. No obvious "TATA" box is seen near the putative transcription start site. However, a CCAAT box is present at nucleotides -89 to -85 similar to its location at nucleotides - 94 to -90 in the murine glut4 promoter. Over a span of 59 bases containing the CCAAT box (-124 to -66) the human sequence is identical to the murine in all but two positions.

Three potential MyoD binding sites occur at nucleotides- 1783 to -1778, -1145 to -1140 and -386 to -381. This sequence, CACCTG, is identical to the high affinity MyoD binding site in the muscle creatine kinase enhancer (Lassar et al., Cell 58: 823-831 [1989]). Three other putative sites possessing the more general CANNTG sequence are also present in the 5' region. These sites possess very similar locations and sequences to sites in the murine promoter (Kaestner et al., Proc. Natl. Acad. Sci. USA 86: 3150-3154 [1990]).

As the human glut4 5'-flanking region possesses MyoD binding sequences with similarity to those found both in the muscle creatine kinase and the murine glut4 genes, it can be hypothesized that at least some of these regions can be utilized to bind MyoD or other myogenic trans-activating factors. They could, therefore, be utilized in the muscle specific activation of human glut4 gene transcription.

Two possible C/EBP binding sites are found at positions -396 to - 389 and -458 to -451. The former sequence matches 6 of 7 bases of the putative C/EBP binding site (TT/GNNGC/TAAG/T) (Ryden and Beemon, Mol. Cell. Biol. 9: 1155-1164 [1989]) and the latter its complement. Expression of this transcription factor, as well as, glut4 has been shown to rise dramatically when 3T3- Ll preadipocytes are differentiated into adipocytes (Birkenmeier et al., Genes Dev. 3: 1145-1156 [1989]). Recently, it has been demonstrated in cotransfection studies that C/EBP transactivates the murine glut4 promoter in 3T3-Ll fibroblasts (Kaestner et. al., 1990). Footprinting studies supported the conclusion that C/EBP was binding to a specific site in the promoter with the appropriate sequence. C/EBP may specifically activate the human glut4 promoter through its interaction with at least one of the potential sites described above, thereby regulating adipocyte-specific expression of the gene.

Two potential insulin responsive elements are located at nucleotides -549 to -543 and -537 to -531. The former is a 7 of 8 base match with the HGAPDH promoter IRE-A core region (Nasrin et al., Proc. Natl. Acad. Sci. USA 87: 5273-5277 [1990]) and the latter a 7 of 8 base match with its reverse complement. Such a site has been shown to mediate, in part, the stimulatory effect of insulin on the HGAPDH promoter activity. Such sites may play a similar role in the human glut4 regulatory region as insulin has been shown to increase expression of glut4 in certain animal models of insulin resistance (Berger et al., Nature 340: 70-72 [1989]; Garvey et al., Science 245: 60-63 [1989]; Kahn et al., J. Clin. Invest. 84: 404-411 [1989]; Sivitz et al., Nature 340: 72-74 [1989]; Strout et al., Endocrinology 126:2728-2732 [1990]).

Four potential SPl binding sites were also found in the 5'-flanking region at nucleotides -301 to -296, -278 to - 273, -165 to -160 and +62 to +67. The first two sites match the SP1 core hexanucleotide sequence GGGCGG (Mitchell and Davis, Science 245: 371-378 [1989]), the latter two match its complement.

EXAMPLE 2

Construction Of An Expression Vector Containing The PCR-DERIVED Human Glut4 Promoter And Protein Expression With The Vector

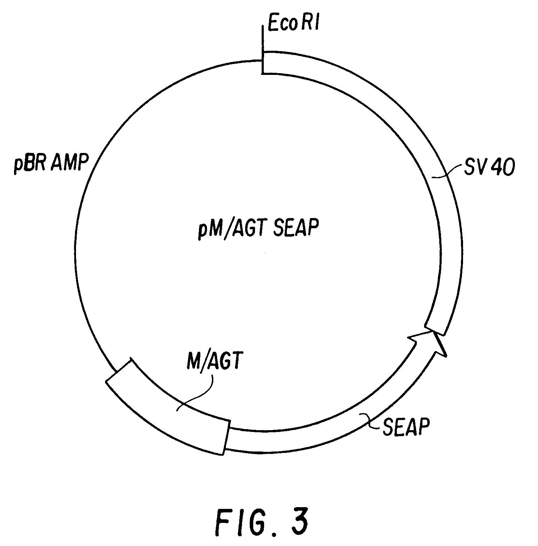

Alter sequencing the 458 bp, PCR-derived glut4 promoter region, it was removed from the SP 72 vector by cleavage within the polylinker region of the vector with restriction endonucleases BmHI and EcoRI. The glut4 promoter region (M/AGT) (Fig. 3) was purified by agarose gel electrophoresis blunt-ended with Klenow DNA polymerase I, and subcloned into the blunt-ended BamHI site of the vector pSV0/SEAP. The pSVOSEAP vector was constructed as follows. The vector pSVOAP (Yoon et al., Gene 66: 11-17 [1988]) was cleaved with Sma I and Hind III to remove the rat alkaline phosphatase cDNA contained therein. The remaining vector pSVO DNA was purified by agarose gel electrophoresis. The vector pBC12/RSV/SEAP (Berger, et al., Gene 66: 1-10 [1988]) was cleaved with Xho I, filled in with Klenow fragment and cleaved with Hind III to remove the c DNA encoding secreted alkaline phosphatase (SEAP). The SEAP cDNA was purified by electrophoresis in agarose gel. The SEAP cDNA was ligated into the Sma I/Hind III site of pSVO to yield pSVOSEAP. A clone was selected in which the which the orientation of the glut4 promoter region was such that it could drive expression of the adjacent secreted alkaline phosphatase gene. This clone was designated pM/AGT/SEAP. A second clone with the glut4 promoter region in the opposing orientation was designated pM/AGT(REV)/SEAP.

Transfection of the chimeric constructs.

The chimeric constructs were transfected into 80% confluent Chinese Hamster Ovary (CHO) cells by calcium phosphate coprecipitation. Five micrograms of CsCl-purified vector was precipitated on a 35-mm dish of cells. After 6 h the cells were shocked with 1% glycerol and washed twice with phosphate buffered saline alter which fresh media containing 10% fetal calf serum was added. Alter an additional 72 hours media was collected from the cells and cell debris was removed by centrifugation. The media was preincubated for 60 min at 65° C and was then assayed for alkaline phosphatase activity as previously described. (Berger et al., Gene 66: 1-10 [1988]). The culture media form cells transfected with pM/AGT/SEAP contained almost ten times more phosphatase activity than those transfected with an equal amount of pM/AGT (REV)/SEAP. This illustrates that the glut4 promoter region, when correctly orientated, is actively driving expression of the secreted alkaline phosphatase gene in CHO cells.

SEQUENCE LISTING

```
    (2)  INFORMATION FOR SEQ ID NO: 1:
       (i) SEQUENCE CHARACTERISTICS:
          (A)  LENGTH:  22 bases
          (B)  TYPE:  nucleic acid
          (C)  STRANDEDNESS:  single
          (D)  TOPOLOGY:  linear
       (ii) MOLECULE TYPE:  genomic DNA
       (iii) HYPOTHETICAL:  yes
       (iv) ANTI-SENSE:  no
       (v) FRAGMENT TYPE:  not applicable
       (vi) ORIGINAL SOURCE:
          (A)  ORGANSIM:  Human
       (vii)  IMMEDIATE SOURCE:
          (A)  LIBRARY:  genomic
          (B)  CLONE:  EMBL 3 SP6T7
       (xi)  SEQUENCE DESCRIPTION: SEQ ID NO:1
       TAATACGACT CACTATAGGG AG  22


    (2)  INFORMATION FOR SEQ ID NO:  2:
       (i) SEQUENCE CHARACTERISTICS:
          (A)  LENGTH:  21 bases
          (B)  TYPE:  nucleic acid
          (C)  STRANDEDNESS:  single
          (D)  TOPOLOGY:  linear
       (ii) MOLECULE TYPE:  genomic DNA
       (iii) HYPOTHETICAL:  yes
```

(iv) ANTI-SENSE:  no

(v) FRAGMENT TYPE:  not applicable

(vi) ORIGINAL SOURCE:

  (A)  ORGANSIM:  Human

(vii) IMMEDIATE SOURCE:

  (A)  LIBRARY:  cDNA

  (B)  CLONE:  lhJHT-3

(ix) FEATURE: not applicable

(x)   PUBLICATION INFORMATION

  (A)   AUTHORS:  Fukumoto, Hirofumi

               Kayano, Toshiaki

               Buse, John B.

               Edwards, Yvonne

               Pilch, Paul F.

               Bell, Graeme I.

               Seino, Susumu

  (B)   TITLE:  Cloning and Characterization of

             the Major Insulin-responsive

             Glucose Transporter Expressed in

             Human Skeletal Muscle and Other

             Insulin-responsive Tissues

  (C)   JOURNAL:  The Journal of Biological

             Chemistry

  (D)   VOLUME:  264

  (E)   ISSUE:  14

  (F)   PAGES:  7776-7779

  (G)   DATE:  May 15, 1989

(xi)  SEQUENCE DESCRIPTION: SEQ ID NO:2

GATCCTGGAG TCTCTGACTC C   21


(2)   INFORMATION FOR SEQ ID NO:  3:

```
(i)  SEQUENCE CHARACTERISTICS:
   (A)  LENGTH:  22 bases
   (B)  TYPE:  nucleic acid
   (C)  STRANDEDNESS:  single
   (D)  TOPOLOGY:  linear
(ii)  MOLECULE TYPE:  genomic DNA
(iii)  HYPOTHETICAL:  yes
(iv)  ANTI-SENSE:  no
(v)  FRAGMENT TYPE:  not applicable
(vi)  ORIGINAL SOURCE:
   (A)  ORGANSIM:  Human
(vii)      IMMEDIATE SOURCE:
   (A)  LIBRARY:  cDNA
   (B)  CLONE:  1hJHT-3
(ix)  FEATURE:  not applicable
(x)          PUBLICATION INFORMATION
   (A)  AUTHORS:  Fukumoto, Hirofumi
                  Kayano, Toshiaki
                  Buse, John B.
                  Edwards, Yvonne
                  Pilch, Paul F.
                  Bell, Graeme I.
                  Seino, Susumu
   (B)  TITLE:  Cloning and Characterization of
               the Major Insulin-responsive
               Glucose Transporter Expressed in
               Human Skeletal Muscle and Other
               Insulin-responsive Tissues
   (C)  JOURNAL:  The Journal of Biological
                  Chemistry
   (D)  VOLUME:  264
   (E)  ISSUE:  14
```

```
(F)   PAGES:  7776-7779
(G)   DATE:  May 15, 1989
(xi)   SEQUENCE DESCRIPTION: SEQ ID NO:3
GCGAAGATGA AAGAACCGAT CC  22


(2) INFORMATION FOR SEQ ID NO: 4:
  (i) SEQUENCE CHARACTERISTICS:
    (A) LENGTH:  2320 base pairs
    (B) TYPE:  nucleic acid
    (C) STRANDEDNESS:  single
    (D) TOPOLOGY:  linear
  (ii) MOLECULE TYPE:  genomic DNA
  (iii) HYPOTHETICAL:  yes
  (iv) ANTI-SENSE:  no
  (v) FRAGMENT TYPE:  not applicable
  (vi) ORIGINAL SOURCE:
    (A)   ORGANSIM:  Human
  (vii)     IMMEDIATE SOURCE:
    (A)   LIBRARY:  genomic
    (B)   CLONE:  EMBL 3 SP6T7
  (ix) FEATURE:  not applicable
  (xi)   SEQUENCE DESCRIPTION: SEQ ID NO:4


GGATCAAGGT CCCCAGAAGC CGGAAACAAA GTGAACCTGT     40
GGAGCAGGAA CTGGGTGAGG AAGCTTTCTT TGAAGGATGA     80
AGAGGAGTCC TTTGGAATTC CGGATTTTTT TTTTTCTTCT     120
TGAGACGGAG TCACTCTGTC GCCAGGCTGG AGTGCAAGGG     160
CACGATCTTG GCTCACTACA ACCTCCACCT CCTGGGTTCA     200
AGCCATTTTC CTGCCTCAGC CTCCCGAGTA GCTGGGATTA     240
CAGGTGTGCA TAACCACGCC CGGCTAATTT TTGTATCTTT     280
AGCAGACATG GGGTTTCTCT ATGTTGGCCA GGCTGGTTTC     320
```

```
AAACTCCTGA CCTCAGTCGA TCCACCTGCC TTGGCCTCCT        360
AAAGTGCTGG GATTACAGGC ATGAGCCACC AGGCCGGGCC        400
GGCATTCCAG ATTTTTCAGG GGATTAGTGC AGCAAAGGAA        440
TCAAGAAGGG ATGTAAAGGC ACAGTGTGTT CTGGGTACAA        480
TAAGGACTTA GGCATTGCCC AGAGCAGGAG GCGAAGGAGA        520
TAGAAGGAGA GGCAGGAGAG ATAGGTAAGG CCAGAGATCG        560
GATAAGAGAG GCAGGAGGTT TTGTTCACTC TGAAAAGGGA        600
TTTGAACTTG GCAATTGGGG CAACAGAGAC AGTGACTTCT        640
TGCTTGAGAG ATGAGATTGG ACCTTCGAAA ATTGTTCTCT        680
GCCCTCGTCA TAAAGGAAAT AAGAGGAGCA CGAAGACCAG        720
TGAGGGTGAT GGTGATCTGG ACTGAAGTGG CAGCCGCCAC        760
GGAGAATATC GGATGAATGT GAGAGAGTTT TGGAGGTCAA        800
AGCACCAATG TTGGAAACTA ACTGGATAAA CGAGGAGAGC        840
GGCGCAGGAC AGGAGGAATC GAGCCTGACT TCTACCATAG        880
GGGTGACTGG GCGGGTAATT CATTGAAATA AGGAAGTTAG        920
GAGGAGGAGC AGGTTTGGAC ATGCTGATCA CTAGAGCTGC        960
CACATCCGGG CGGTAACGAA CACCTGGATC TGCAGCTCCA       1000
GAGAAGGGCC TGGGTCAGAT GTCACTGAAG CCCTATGGTG       1040
GCGGAAAGGC GAGAAATAGT GGGTTGAGAT TCCAAGTGCA       1080
ATCCACTGCG GCTCCTCGCT CGCCCTCCAG GTGGCAGCAC       1120
AACCCTGCGC TTCCGAAGCC CGTTTTCTGA GCCAGACACT       1160
CTCCACGCTC TGGGTATTTC GGCTTCTCTC TCCCCACACG       1200
CCGACCCTAG GTCGCGCACT TTCTGCCTGG CAGAATTTGG       1240
CCGAGGATCC AAACCCGGAG CAGCCTCCAG AGAGCGTGTC       1280
GTTCACGCGG CCAGCATATG CTCAGAGACC TCAGAGGCTC       1320
AGAGACCTCA GGGCTGGTGG TGTGGTCGGT TGTGACCACT       1360
TGTCCCTCGG ACCGGCTCCA GGAACCAACC TGGGGAATGT       1400
GTGTAGGGGA AGGGCGGGAT AGACAGTGCC CGGAGCAGGG       1440
AGGCGCTGAA AGACAGGACC AAGCAGCCCG GCCACCAGAC       1480
CCGTTGTGGG AACGGAATTT CCTGGCCCCC AGGGCCACAC       1520
TCGCGTGGGA AGCATGTCGC GGACCCTTTA AGGCGTCATC       1560
TCCCTGTCTC TCCGCCCCCG CCTGGGACAG GCGGGACGCC       1600
```

```
CGGGACCTGA CATTTGGAGG CTCCCAACGT GGGAGCTAAA     1640

AATAGCAGCC CCGGGTTACT TTGGGGCATT GCTCCTCTCC     1680

CAACCCGCGC GCCGGCTCGC GAGCCGTCTC AGGCCGCTGG     1720

AGTTTCCCCG GGGCAAGTAC ACCTGGCCCG TCCTCTCCTC     1760

TCAGACCCCA CTGTCCAGAC CCGCAGAGTT TAAGATGCTT     1800

CTGCAGCCCG GGATCCTAGC TGGTGGGCGG AGTCCTAACA     1840

CGTGGGTGGG CGGGGCCTTT TGTTCCAGGG ACTCTTTTCT     1880

CAAAACTTCC CAGTCGGAGG CTGGCGGGAA CCCGAGAGGC     1920

GTGTCTCGCC AGCCACGCGG AGGGGCGTGG CCTCATTGGC     1960

CCGCCCCACC AACTCCAGCC AAACTCTAAA CCCCAGGCGG     2000

AGGGGGCGTG GCCTTCTGGG GTGTGCGGGC TCCTGGCCAA     2040

TGGGTGCTGT GAAGGGCGTG GCCCGCGGGG GCAGGAGCGA     2080

GGTGGCGGGG GCTTCTCGCG TCTTTTCCCC CAGCCCCGCT     2120

CCACCAGATC CGCGGGAGCC CCACTGCTCT CCGGGTCCTT     2160

GGCTTGTGGC TGTGGGTCCC ATCGGGCCCG CCCTCGCACG     2200

TCACTCCGGG ACCCCCGCGG CCTCCGCAGG TTCTGCGCTC     2240

CAGGCCGGAG TCAGAGACTC CAGGATCGGT TCTTTCATCT     2280

TCGCCGCCCC TGCGTCCAGC TCTTCTAAGA CGAGATGATG     2320
```

Claims

1. A substantially purified segment of DNA comprising a human glut4 promoter.

2. The human glut4 promoter DNA of claim 1 wherein said promoter when operably attached to a structural gene will initiate transcription of said structural gene.

3. A substantially purified segment of human DNA comprising a functionally operable glut4 promoter sequence capable of promoting the expression of a heterologous gene with said promoter sequence located upstream from and proximal to the transcription initiation site of the heterologous gene and with said promoter sequence being free of the homologous gene ordinarily under the transcriptional control of said promoter sequence.

4. A substantially purified segment of DNA having a nucleotide sequence of:

```
GGATCAAGGT CCCCAGAAGC CGGAAACAAA GTGAACCTGT

GGAGCAGGAA CTGGGTGAGG AAGCTTTCTT TGAAGGATGA

AGAGGAGTCC TTTGGAATTC CGGATTTTTT TTTTTCTTCT

TGAGACGGAG TCACTCTGTC GCCAGGCTGG AGTGCAAGGG

CACGATCTTG GCTCACTACA ACCTCCACCT CCTGGGTTCA

AGCCATTTTC CTGCCTCAGC CTCCCGAGTA GCTGGGATTA

CAGGTGTGCA TAACCACGCC CGGCTAATTT TTGTATCTTT

AGCAGACATG GGGTTTCTCT ATGTTGGCCA GGCTGGTTTC
```

```
AAACTCCTGA CCTCAGTCGA TCCACCTGCC TTGGCCTCCT

AAAGTGCTGG GATTACAGGC ATGAGCCACC AGGCCGGGCC

GGCATTCCAG ATTTTTCAGG GGATTAGTGC AGCAAAGGAA

TCAAGAAGGG ATGTAAAGGC ACAGTGTGTT CTGGGTACAA

TAAGGACTTA GGCATTGCCC AGAGCAGGAG GCGAAGGAGA

TAGAAGGAGA GGCAGGAGAG ATAGGTAAGG CCAGAGATCG

GATAAGAGAG GCAGGAGGTT TTGTTCACTC TGAAAAGGGA

TTTGAACTTG GCAATTGGGG CAACAGAGAC AGTGACTTCT

TGCTTGAGAG ATGAGATTGG ACCTTCGAAA ATTGTTCTCT

GCCCTCGTCA TAAAGGAAAT AAGAGGAGCA CGAAGACCAG

TGAGGGTGAT GGTGATCTGG ACTGAAGTGG CAGCCGCCAC

GGAGAATATC GGATGAATGT GAGAGAGTTT TGGAGGTCAA

AGCACCAATG TTGGAAACTA ACTGGATAAA CGAGGAGAGC

GGCGCAGGAC AGGAGGAATC GAGCCTGACT TCTACCATAG

GGGTGACTGG GCGGGTAATT CATTGAAATA AGGAAGTTAG

GAGGAGGAGC AGGTTTGGAC ATGCTGATCA CTAGAGCTGC

CACATCCGGG CGGTAACGAA CACCTGGATC TGCAGCTCCA

GAGAAGGGCC TGGGTCAGAT GTCACTGAAG CCCTATGGTG

GCGGAAAGGC GAGAAATAGT GGGTTGAGAT TCCAAGTGCA

ATCCACTGCG GCTCCTCGCT CGCCCTCCAG GTGGCAGCAC

AACCCTGCGC TTCCGAAGCC CGTTTTCTGA GCCAGACACT

CTCCACGCTC TGGGTATTTC GGCTTCTCTC TCCCCACACG

CCGACCCTAG GTCGCGCACT TTCTGCCTGG CAGAATTTGG
```

16

CCGAGGATCC AAACCCGGAG CAGCCTCCAG AGAGCGTGTC

GTTCACGCGG CCAGCATATG CTCAGAGACC TCAGAGGCTC

AGAGACCTCA GGGCTGGTGG TGTGGTCGGT TGTGACCACT

TGTCCCTCGG ACCGGCTCCA GGAACCAACC TGGGGAATGT

GTGTAGGGGA AGGGCGGGAT AGACAGTGCC CGGAGCAGGG

AGGCGCTGAA AGACAGGACC AAGCAGCCCG GCCACCAGAC

CCGTTGTGGG AACGGAATTT CCTGGCCCCC AGGGCCACAC

TCGCGTGGGA AGCATGTCGC GGACCCTTTA AGGCGTCATC

TCCCTGTCTC TCCGCCCCCG CCTGGGACAG GCGGGACGCC

CGGGACCTGA CATTTGGAGG CTCCCAACGT GGGAGCTAAA

AATAGCAGCC CCGGGTTACT TTGGGGCATT GCTCCTCTCC

CAACCCGCGC GCCGGCTCGC GAGCCGTCTC AGGCCGCTGG

AGTTTCCCCG GGGCAAGTAC ACCTGGCCCG TCCTCTCCTC

TCAGACCCCA CTGTCCAGAC CCGCAGAGTT TAAGATGCTT

CTGCAGCCCG GGATCCTAGC TGGTGGGCGG AGTCCTAACA

CGTGGGTGGG CGGGGCCTTT TGTTCCAGGG ACTCTTTTCT

CAAAACTTCC CAGTCGGAGG CTGGCGGGAA CCCGAGAGGC

GTGTCTCGCC AGCCACGCGG AGGGGCGTGG CCTCATTGGC

CCGCCCCACC AACTCCAGCC AAACTCTAAA CCCCAGGCGG

AGGGGGCGTG GCCTTCTGGG GTGTGCGGGC TCCTGGCCAA

TGGGTGCTGT GAAGGGCGTG GCCCGCGGGG GCAGGAGCGA

GGTGGCGGGG GCTTCTCGCG TCTTTTCCCC CAGCCCCGCT

CCACCAGATC CGCGGGAGCC CCACTGCTCT CCGGGTCCTT


GGCTTGTGGC TGTGGGTCCC ATCGGGCCCG CCCTCGCACG

TCACTCCGGG ACCCCCGCGG CCTCCGCAGG TTCTGCGCTC

CAGGCCGGAG TCAGAGACTC CAGGATCGGT TCTTTCATCT

TCGCCGCCCC TGCGTCCAGC TCTTCTAAGA CGAG

with said sequence being capable of promoting the expression of a heterologous gene, with said promoter sequence located upstream from and proximal to the transcription initiation site of the heterologous gene and with said promoter sequence being free of the homologous gene ordinarily under the transcriptional control of said promoter sequence. (SEQ ID NO: 4)

5. A substantially purified segment of DNA having a nucleotide sequence of:

```
GATCCTAGC TGGTGGGCGG AGTCCTAACA

CGTGGGTGGG CGGGGCCTTT TGTTCCAGGG ACTCTTTTCT

CAAAACTTCC CAGTCGGAGG CTGGCGGGAA CCCGAGAGGC

GTGTCTCGCC AGCCACGCGG AGGGGCGTGG CCTCATTGGC

CCGCCCCACC AACTCCAGCC AAACTCTAAA CCCCAGGCGG

AGGGGGCGTG GCCTTCTGGG GTGTGCGGGC TCCTGGCCAA

TGGGTGCTGT GAAGGGCGTG GCCCGCGGGG GCAGGAGCGA

GGTGGCGGGG GCTTCTCGCG TCTTTTCCCC CAGCCCCGCT

CCACCAGATC CGCGGGAGCC CCACTGCTCT CCGGGTCCTT

GGCTTGTGGC TGTGGGTCCC ATCGGGCCCG CCCTCGCACG


TCACTCCGGG ACCCCCGCGG CCTCCGCAGG TTCTGCGCTC

CAGGCCGGAG TCAGAGACTC CAGGATC
```

with said sequence being capable of promoting the expression of a heterologous gene, with said promoter sequence located upstream from and proximal to the transcription initiation site of the heterologous gene and with said promoter sequence being free of the homologous gene ordinarily under the transcriptional control of said promoter sequence. (SEQ ID NO: 4)

6. A substantially purified segment of DNA comprising a human glut4 promoter in combination with a selected heterologous structural gene, said structural gene and promoter being combined in a manner such that said structural gene is under the transcriptional control of said human glut4 promoter.

7. A recombinant DNA vector comprising a DNA sequence as defined by claim 3.

8. A mammalian cell comprising a recombinant DNA vector as defined by claim 7.

9. A recombinant DNA vector comprising a DNA sequence as defined by claim 4.

10. A mammalian cell comprising a recombinant DNA vector as defined by claim 9.

11. A method for expressing a selected structural gene comprising the following:
   a. transforming a cell with a segment of DNA, which segment includes a glut4 promoter DNA sequence as defined in claim 3 in combination with a selected heterologous structural gene, said structural gene and promoter being combined in a manner such that said structural gene is under the transcriptional control of said glut4 promoter; and
   b. culturing the transformed cells under conditions appropriate for expression of the heterologous gene which results in the production of the heterologous gene product.

FIG. 1

**FIG.2A**

```
-2125  GGATCAAGGTCCCCAGAAGCCGGAAACAAAGTGAACCTGTGGAGCAGGAACTGGGTGAGG
-2065  AAGCTTTCTTTGAAGGATGAAGAGGAGTCCTTTGGAATTCCGGATTTTTTTTTCTTCT
-2005  TGAGACGGAGTCACTCTGTCGCCAGGCTGGAGTGCAAGGGCACGATCCTTGGCTCACTACA
-1945  ACCTCCACCTCCTGGGTTCAAGCCATTTTCCTGCCTCAGCCTCCCGAGTAGCTGGGATTA
-1885  CAGGTGTGCATAACCACGCCCGGCTAATTTTTGTATCTTTAGCAGACATGGGTTTCTCT
-1825  ATGTTGGCCAGGCTGGTTTCAAACTCCTGACCTCAGTCGATCCACCTGCCTTGGCCTCCT
-1765  AAAGTGCTGGGATTACAGGCATGAGCCACCAGGCCCGGGCCATTCCAGATTTTTCAGG
-1705  GGATTAGTGCAGCAAAGGAATCAAGAGAATGTAAAGGCACAGTGTGTTCTGGGTACAA
-1645  TAAGGACTTAGGCATTGCCCAGAGCAGGAGGCGAAGGAGATAGAAGGAGAGGCAGGAGAG
-1585  ATAGGTAAGGCCCAGAGATCGGATAAGAGAGGCAGGAGGTTTGTTCACTCTGAAAAGGGA
-1525  TTTGAACTTGGCAATTGGGGCAACAGAGACAGTGACTTCTTGCTTGAGAGATGAGATTGG
-1465  ACCTTCGAAAATTGTTCTCTGCCCTCGTCATAAAGGAAATAAGAGGAGCACGAAGACCAG
-1405  TGAGGGTGATGGTGATCTGGACTGAAGTGTCAAAGCACCAATGTTGGAAACTGGATGAATGT
-1345  GAGAGAGTTTGGAGGTCAAAGCACCAATGTTGGAAACTAACTGGATAAACGAGGAGAGC
-1285  GGCGCAGGACAGGAGGAATCGAGCCTGACTTCTACCATAGGGGTGACTGGGCGGGTAATT
```

-1225 CATTGAAATAAGGAAGTTAGGAGGAGGAGCAGGTTTGGACATGCTGATCACTAGAGCTGC

-1165 CACATCCGGGCGGTAACGAACACCTGGATCTGCAGCTCCAGAGAAGGGCCTGGGTCAGAT

-1105 GTCACTGAAGCCCTATGGTGGCGGAAAGGCGAGAAATAGTGGGTTGAGATTCCAAGTGCA

-1045 ATCCACTGCGGCTCCTCGCTCGCCCTCCAGGTGGCAGCACAACCCTGCGCTTCCGAAGCC

-985 CGTTTTCTGAGCCAGACACTCTCCACGCTCTGGGTATTTCGGCTTCTCTCTCCCCACACG

-925 CCGACCCTAGGTCGCGCACTTTCTGCCTGGCAGAATTTGGCCGAGGATCCAAACCCGGAG

-865 CAGCCTCCAGAGAGCGTGTCGTTCACGCGGCCAGCATATGCTCAGAGACCTCAGAGGCTC

-805 AGAGACCTCAGGGCTGGTGGTGTGGTCGGTTGTGACCACTTGTCCCTCGGACCGGCTCCA

-745 GGAACCAACCTGGGGAATGTGTGTAGGGGAAGGGCGGGATAGACAGTGCCCGGAGCAGGG

-685 AGGCGCTGAAAGACAGGACCAAGCAGCCCGGCCACCAGACCCGTTGTGGGAACGGAATTT

-625 CCTGGCCCCCAGGGCCACACTCGCGTGGGAAGCATGTCGCGGACCCTTTAAGGCGTCATC

-565 TCCCTGTCTCTCCGCCCCCGCCTGGGACAGGCGGGACGCCCGGGACCTGACATTTGGAGG

FIG. 2B

-505 CTCCCAACGTGGGAGCTAAAAATAGCAGCCCCGGGTTACTTTGGGGCATTGCTCCTCTCC

-445 CAACCCGCGCGCCGGCTCGCGAGCCGTCTCAGGCCGCTGGAGTTTCCCCGGGGCAAGTAC

-385 ACCTGGCCCGTCCTCTCCTCTCAGACCCCACTGTCCAGACCCGCAGAGTTTAAGATGCTT

EP 0 496 453 A1

EP 0 496 453 A1

-325  CTGCAGCCCGGGATCCTAGCTGGTGGGCGGAGTCCTAACACGTGGGTGGGCGGGGCCTTT

-265  TGTTCCAGGGACTCTTTTCTCAAAACTTCCCAGTCGGAGGCTGGCGGGAACCCGAGAGGC

-205  GTGTCTCGCCAGCCACGCGGAGGGGCGTGGCCTCATTGGCCCGCCCCACCAACTCCAGCC

-145  AAACTCTAAACCCCAGGCGGAGGGGGCGTGGCCTTCTGGGGTGTGCGGGCTCCTGGCCAA

-85   TGGGTGCTGTGAAGGGCGTGGCCCGCGGGGGCAGGAGCGAGGTGGCGGGGGCTTCTCGCG

-25   TCTTTTCCCCCAGCCCCGCTCCACCAGATCCGCGGGAGCCCCACTGCTCTCCGGGTCCTT

+36   GGCTTGTGGCTGTGGGTCCCATCGGGCCCGCCCTCGCACGTCACTCCGGGACCCCCGCGG

+96   CCTCCGCAGGTTCTGCGCTCCAGGCCGGAGTCAGAGACTCCAGGATCGGTTCTTTCATCT

+156  TCGCCGCCCCTGCGTCCAGCTCTTCTAAGACGAGATG

# FIG. 2C

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | PROCEEDINGS OF THE NATIONAL ACADEMY OF SCIENCES OF USA. vol. 87, no. 1, January 1990, WASHINGTON US pages 251 - 255; K.H. KAESTNER ET AL.: 'Mouse insulin-responsive glucose transporter gene: Characterization of the gene and trans-activation by the CCAAT/enhancer binding protein' * Whole article * --- | 1-11 | C12N15/85 C12N15/62 C12N15/12 C07K15/00 |
| D,Y | JOURNAL OF BIOLOGICAL CHEMISTRY vol. 264, no. 14, 15 May 1989, BALTIMORE, US pages 7776 - 7779; H. FUKUMOTO ET AL.: 'Cloning and characterization of the major insulin-responsive glucose transporter expressed in human skeletal muscle and other insulin-responsive tissues' * Whole article * --- | 1-11 | |
| D,A | PROCEEDINGS OF THE NATIONAL ACADEMY OF SCIENCES OF USA. vol. 86, no. 9, May 1989, WASHINGTON US pages 3150 - 3154; K.H. KAESTNER ET AL.: 'Sequence, tissue distribution, and differential expression of mRNA for a putative insulin-responsive glucose transporter in mouse 3T3-L1 adipocytes' * Whole article * --- | 1 | **TECHNICAL FIELDS SEARCHED (Int. Cl.5 )** C12N C07K |
| Y | DIABETES vol. 37, no. 5, May 1988, NEW YORK, US pages 657 - 661; H. FUKUMOTO ET AL.: 'Characterization and expression of human HepG2/erythrocyte glucose-transporter gene' * Whole article, in particular page 660* --- -/-- | 1-11 | |

The present search report has been drawn up for all claims

| Place of search | Date of completion of the search | Examiner |
|---|---|---|
| BERLIN | 07 APRIL 1992 | JULIA P. |

EPO FORM 1503 03.82 (P0401)

European Patent
Office

EUROPEAN SEARCH REPORT

Application Number

EP    92 20 0112
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DIABETES CARE<br>vol. 13, no. 6, June 1990, NEW YORK, US<br>pages 565 - 575;<br>M.D. LANE ET AL.: 'Insulin-receptor tyrosine kinase and glucose transport'<br>* Abstract,  pages 572 - 574 *<br>--- | 1-11 | |
| A | FASEB (FED. AM. SOC. FOR EXP. BIOL.) JOURNAL<br>vol. 4, no. 7, 26 April 1990, WASHINGTON, US<br>page A1905;<br>K.H. KAESTNER ET AL.: 'Mouse insulin-responsive glucose transporter gene: Characterization of the gene and transactivation by c/EBP'<br>* Abstract *<br>--- | 1-11 | |
| D,A | DIABETES CARE<br>vol. 13, no. 3, March 1990, NEW YORK, US<br>pages 198 - 208;<br>G.I. BELL ET AL.: 'Molecular biology of mammmalian glucose transporters'<br>* Whole article *<br><br>----- | 1 | TECHNICAL FIELDS SEARCHED (Int. Cl.5) |

The present search report has been drawn up for all claims

| Place of search | Date of completion of the search | Examiner |
|---|---|---|
| BERLIN | 07 APRIL 1992 | JULIA P. |